(19) **Europäisches Patentamt**
**European Patent Office**
**Office européen des brevets**

(11) **EP 4 458 253 B1**

(12) **EUROPEAN PATENT SPECIFICATION**

(45) Date of publication and mention
of the grant of the patent:
**22.07.2026 Bulletin 2026/30**

(21) Application number: **22916596.4**

(22) Date of filing: **21.12.2022**

(51) International Patent Classification (IPC):
**A61B 5/00** *(2006.01)*      **A61B 5/11** *(2006.01)*
**A61B 5/024** *(2006.01)*      **A61B 5/08** *(2006.01)*
**G16H 50/20** *(2018.01)*

(52) Cooperative Patent Classification (CPC):
**A61B 5/0077; A61B 5/02416; A61B 5/0816;**
**A61B 5/1102; A61B 5/7485;** A61B 2503/04

(86) International application number:
**PCT/KR2022/020971**

(87) International publication number:
**WO 2023/128454 (06.07.2023 Gazette 2023/27)**

(54) **DIGITAL HEALTHCARE DEVICE FOR MEASURING HEART RATE USING REMOTE PPG**

DIGITALE GESUNDHEITSVORRICHTUNG ZUR MESSUNG DER HERZFREQUENZ MITTELS
FERN-PPG

DISPOSITIF DE SOINS DE SANTÉ NUMÉRIQUE PERMETTANT DE MESURER LA FRÉQUENCE
CARDIAQUE À L'AIDE D'UNE PHOTOPLÉTHYSMOGRAPHIE À DISTANCE

(84) Designated Contracting States:
**AL AT BE BG CH CY CZ DE DK EE ES FI FR GB**
**GR HR HU IE IS IT LI LT LU LV MC ME MK MT NL**
**NO PL PT RO RS SE SI SK SM TR**

(30) Priority: **30.12.2021 KR 20210192187**
**15.04.2022 KR 20220046601**

(43) Date of publication of application:
**06.11.2024 Bulletin 2024/45**

(73) Proprietor: **Emma Healthcare Co., Ltd.**
**Seongnam-si, Gyeonggi-do 13503 (KR)**

(72) Inventor: **SOHN, Ryang Hee**
**Seongnam-si Gyeonggi-do 13503 (KR)**

(74) Representative: **Schmitt-Nilson Schraud Waibel**
**Wohlfrom**
**Patentanwälte Partnerschaft mbB**
**Pelkovenstraße 143**
**80992 München (DE)**

(56) References cited:
EP-A1- 3 838 128      KR-A- 20140 058 573
KR-A- 20190 007 803      KR-A- 20200 059 584
KR-A- 20210 047 022      KR-A- 20210 085 867

KR-B1- 102 416 878      US-A1- 2016 206 216
US-A1- 2020 156 648      US-A1- 2021 201 496

- SCEBBA GAETANO ET AL: "Improving ROI
detection in photoplethysmographic imaging
with thermal cameras", 2017 39TH ANNUAL
INTERNATIONAL CONFERENCE OF THE IEEE
ENGINEERING IN MEDICINE AND BIOLOGY
SOCIETY (EMBC), IEEE, 11 July 2017
(2017-07-11), pages 4285 - 4288, XP033152972,
DOI: 10.1109/EMBC.2017.8037803
- FARRUKH HABIBA HFARRUKH@PURDUE EDU
ET AL: "FaceRevelio a face liveness detection
system for smartphones with a single front
camera", PROCEEDINGS OF THE 26TH ANNUAL
INTERNATIONAL CONFERENCE ON MOBILE
COMPUTING AND NETWORKING, ACMPUB27,
NEW YORK, NY, USA, 21 September 2020
(2020-09-21), pages 1 - 13, XP058479959, ISBN:
978-1-4503-7085-1, DOI: 10.1145/
3372224.3419206

Note: Within nine months of the publication of the mention of the grant of the European patent in the European Patent
Bulletin, any person may give notice to the European Patent Office of opposition to that patent, in accordance with the
Implementing Regulations. Notice of opposition shall not be deemed to have been filed until the opposition fee has been
paid. (Art. 99(1) European Patent Convention).

**(Cont. next page)**

- LIU YIMING ET AL: "Motion-Robust Multimodal Heart Rate Estimation Using BCG Fused Remote-PPG With Deep Facial ROI Tracker and Pose Constrained Kalman Filter", IEEE TRANSACTIONS ON INSTRUMENTATION AND MEASUREMENT, IEEE, USA, vol. 70, 19 February 2021 (2021-02-19), pages 1 - 15, XP011843151, ISSN: 0018-9456, [retrieved on 20210303], DOI: 10.1109/TIM.2021.3060572

- JI YERIM, LIM SEOYEON, PARK SOYEON, KIM SANGHA, DONG SUH-YEON: "Deep Learning-based Real-time Heart Rate Measurement System Using Mobile Facial Videos", JOURNAL OF KOREA MULTIMEDIA SOCIETY, KOREA MULTIMEDIA SOCIETY, KR, vol. 24, no. 11, 1 November 2021 (2021-11-01), KR
, pages 1481 - 1491, XP093076151, ISSN: 1229-7771, DOI: 10.9717/kmms.2021.24.11.1481

**Description**

[Technical Field]

The present invention relates to a healthcare apparatus, and more particularly to an artificial intelligence (AI)-based digital healthcare apparatus capable of predicting the heart rate of a subject in a contactless manner.

[Background Art]

Research to sense a biometric signal using a PPG sensor in a non-invasive manner to treat human diseases has been conducted. Due to the current COVID-19 pandemic, however, technology capable of remotely monitoring health in a non-invasive manner has become quite important. Many countries have recommended using remote health strategies if possible in order to reduce a danger of COVID-19 in a medical environment. For this reason, a new method other than a biometric information monitoring method through a conventional body contact type sensor is required.

Remote health monitoring technology is based on a communication system, such as a mobile phone or an online health portal. The remote health monitoring technology may be greatly required for continuous patient monitoring even after an epidemic, such as COVID-19, ends. This technology may be used to measure a physiological signal of a user based on a facial video stream using a camera. This technology may also be used for monitoring of biometric information of an infant, monitoring of health of an elder, or mental health monitoring as well as contagious diseases.

However, research to predict the heart rate, the stress index, and the respiratory rate of a person by estimating a biometric signal of the person, as remote health monitoring technology, and a healthcare product based thereon have not yet been proposed. The present invention was developed in a project to develop a smart cradle compatible with wheelchairs, a research and development project for assistive devices for the elderly and disabled supported by the Ministry of Health and Welfare and the Korea Health Industry Development Institute (Project identification number (1465036599), project number (HJ21C0014030022), research period (2022.01.01~) It was calculated with the support of 2022.12.31)).

Scebba Gaetano et al: "Improving ROI detection in photoplethysmographic imaging with thermal cameras", 2017 39th Annual International Conference of the IEEE Engineering in Medicine and Biology Society (EMBC), 20170711 IEEE, is a disclosure relevant to the field of the invention.

In US 2016/206216 A1 a device, system and method for skin detection are presented. The proposed device comprises a thermal sensor input for obtaining thermal sensor data of a scene, a light sensor input for obtaining light sensor data of the scene, and an evaluation unit for analyzing the obtained thermal sensor data and the obtained light sensor data and for detecting skin areas within the scene based on said analysis. Other documents relevant to the field of the invention are found in US 2021/201496 A1, EP 3 838 128 A1, and KR 2014 0058573 A. LIU YIMING ET AL: "Motion-Robust Multimodal Heart Rate Estimation Using BCG Fused Remote-PPG With Deep Facial ROI Tracker and Pose Constrained Kalman Filter",IEEE TRANSACTIONS ON INSTRUMENTATION AND MEASUREMENT, vol. 70, 19 February 2021, pages 1-15, discloses a heart-rate monitoring system comprising a camera configured to obtain facial images for rPPG and a ballistocardiogram (BCG) sensor.

[Disclosure]

[Technical Problem]

The invention is laid out in the subject-matter of the independent claims. Advantageous embodiments are laid out in the dependent claims.

[Technical Solution]

The present disclosure provides a digital healthcare apparatus including a processor configured to determine whether to obtain a face of a subject as a color image or an infrared (IR) image based on it is a predetermined time or a level of ambient light is less than a predetermined level; and a camera configured to obtain a facial image by photographing a face of the subject based on the determination of the processor, wherein the processor is configured to detect a region of interest (ROI) corresponding to the face in the facial image, obtain a first image for a first area and a second image for a second area in the detected ROI, and to output a remote photoplethysmography (rPPG) signal waveform of the subject and a respiratory rate signal waveform by applying the first image and the second image to a predetermined trained algorithm model. The first is a forehead area and the second area is a cheek area.

The digital healthcare apparatus includes ballistocardiogram (BCG) sensor configured to sense a BCG signal of a subject, the processor is configured to calculate a first heart rate from the sensed BCG signal waveform, calculate a second heart rate from the output rPPG signal waveform, and to output a heart rate of the subject based on the first heart rate and the second heart rate. The output heart rate corresponds to an average of the first heart rate and the second heart rate. The predetermined trained algorithm model uses a Siamese Neural Network (SNN) of multi-task learning. The processor is configured to detect two eye area images in the ROI and two pupil images in the detected two eye area images, and determine that the subject is in a wake state when two irises are detected and recognized from the detected two pupil images. The processor may determine that the subject is in a sleep state when both the two irises are not recognized from the detected two pupil images for a predetermined time. The digital healthcare apparatus further comprises a bed configured to allow an infant corresponding to the subject to lie down thereon, the processor may perform control such that a bounce function that is being performed by the bed is maintained upon determining that the subject is in the wake state. The digital healthcare apparatus further comprises a bed configured to allow the subject to lie down thereon, the processor may control vertical and horizontal movements of the bed such that a bounce function is slowly stopped upon determining that the subject is in the sleep state.

In accordance with another aspect, there is provided a monitoring method for healthcare, the monitoring method includes determining whether to obtain a face of the subject as a color image or an infrared (IR) image based on it is a predetermined time or a level of ambient light is less than a predetermined level; and obtaining a facial image by photographing a face of the subject based on the determination of the processor; detecting a region of interest (ROI) corresponding to the face in the facial image; obtaining a first image for a first area and a second image for a second area in the detected ROI; and outputting a remote photoplethysmography (rPPG) signal waveform of the subject and a respiratory rate signal waveform by applying the first image and the second image to a predetermined trained algorithm model. The first is a forehead area and the second area is a cheek area.

The method may include sensing a ballistocardiogram (BCG) signal of the subject; calculating a first heart rate from the sensed BCG signal waveform; calculating a second heart rate from the output rPPG signal waveform; and outputting a heart rate of the subject based on the first heart rate and the second heart rate. The method may further include detecting two eye area images in the ROI and two pupil images in the detected two eye area images, determining that the subject is in a wake state when two irises are detected and recognized from the detected two pupil images. The method may further include determining that the subject is in a sleep state when both the two irises are not recognized from the detected two pupil images for a predetermined time. The method may further include calculating a respiratory rate based on the output respiratory rate signal waveform; and determining whether the subject is in a sleep apnea state based on the calculated respiratory rate.

[Advantageous Effects]

The healthcare device according to one embodiment measures information about the subject's heart rate, respiratory rate, stress index, sleep state (sleep apnea), etc. in a non-contact manner, but can significantly improve accuracy.

The healthcare device according to an embodiment can significantly improve the accuracy of determining wake/sleep status by recognizing the subject's iris.

The healthcare device according to an embodiment enables continuous health monitoring by estimating biosignals by predicting PPG and respiratory rate (RR) of infants, the elderly, and the diseased based on facial images.

The healthcare device according to an embodiment is effective in monitoring the health of infants, patients, etc. by estimating biosignals based on images in a non-contact manner in a situation where an infectious disease such as COVID-19 continues.

The effects that can be obtained are not limited to the effects mentioned above, and other effects not mentioned can be clearly understood by those skilled in the art from the description below.

[Description of Drawings]

The accompanying drawings illustrate embodiment(s) of the invention and together with the description serve to explain the principle of the invention. In the drawings:

FIG. 1 is a view illustrating a layered structure of an artificial neural network;
FIG. 2 is a view illustrating a ballistocardiogram (signal) waveform;
FIG. 3 is an illustrative view provided to explain remote PPG (rPPG);
FIG. 4 is a block diagram provided to explain the construction of a healthcare apparatus 400;
FIG. 5 is a view provided to explain output of a PPG signal and a respiratory rate (RR) signal from an MTL algorithm learning model using a remote PPG technique;
FIG. 6 is a view illustrating PPG signal frequency analysis and LF score/HF score distribution for calculation of a stress index of a subject;

FIG. 7 is a view illustrating the healthcare apparatus 400;

FIG. 8 is a view illustrating a step of detecting the iris of the subject in the healthcare apparatus 400; and

FIG. 9 is a view describing in detail a method of detecting the pupil (iris) of the subject in the healthcare apparatus 400.

[Best Mode]

Hereinafter, preferred embodiments will be described in detail with reference to the accompanying drawings. The detailed description disclosed hereinafter together with the accompanying drawings shows exemplary embodiments. The following detailed description includes specific details in order to provide complete understanding of the present invention.

In some cases, a well-known structure and apparatus may be omitted, or each structure and apparatus will be shown in the form of a block diagram including core functions thereof. In addition, the same elements are denoted by the same reference numerals throughout this specification.

Before describing the present invention, artificial intelligence (AI), machine learning, and deep learning will be described. As a method of most easily understanding the relationship among the three concepts, three concentric circles may be imagined. Artificial intelligence may be the outermost circle, machine learning may be the middle circle, and deep learning, which leads a current artificial intelligence boom, may be the innermost circle.

Hereinafter, deep learning will be described in more detail.

Deep learning, which is a kind of artificial neural network (ANN) using a human neural network theory, is a machine learning model or an algorithm set referring to a deep neural network (DNN) configured to have a layered structure in which at least one hidden layer (hereinafter referred to as an "intermediate layer") is provided between an input layer and an output layer. Briefly, deep learning may be an artificial neural network having deep layers.

A deep neural network, which is a descendant of the artificial neural network, is the latest version of the artificial neural network that goes beyond the existing limits and has achieved successes in areas in which a large number of artificial intelligence technologies suffered failures in the past. When describing modeling an artificial neural network by imitating a biological neural network, biological neurons are modeled as nodes in terms of processing units, and synapses are modeled as weights in terms of connections, as shown in Table 1 below.

[Table 1]

| Biological neural network | Artificial neural network |
| --- | --- |
| Cell body | Node |
| Dendrite | Input |
| Axon | Output |
| Synapse | Weight |

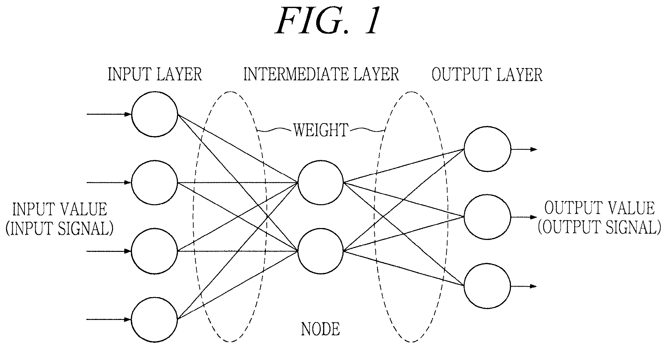

FIG. 1 is a view illustrating a layered structure of an artificial neural network. Like a plurality of biological neurons of a human being, not a single biological neuron, is connected to each other in order to perform a meaningful task, for an artificial neural network, individual neurons are also connected to each other via synapses, whereby a plurality of layers is connected to each other, wherein connection intensity between the respective layers may be updated using weights. The multilayered structure and connection intensity are utilized in a field for learning and recognition. The respective nodes are connected to each other via links having weights, and the entire model performs learning while repeatedly adjusting weights. The weights, which are basic means for long-term memory, express importance of the respective nodes. The artificial neural network initializes the weights and updates and adjusts weights using a data set to be trained in order to train the entire model. When a new input value is input after training is completed, an appropriate output value is inferred. The learning principle of the artificial neural network is a process in which intelligence is formed through generalization of experiences, and learning is performed in a bottom-up manner. When two or more (i.e. 5 to 10) intermediate layers are provided, as shown in FIG. 1, this means that the layers are deepened and is called a deep neural network, and a learning and inference model achieved through the deep neural network may be referred to as deep learning. The artificial neural network may play a role to some extent even when the artificial neural network has one intermediate layer (generally referred to as a "hidden layer") excluding input and output. When problem complexity increases, however, the number of nodes or the number of layers must be increased. It is effective to increase the number of layers so as to provide a multilayered model; however, an available range is restrictive due to limitations in that efficient learning is impossible and the amount of calculation necessary to train the network is large.

Previous deep neural networks were generally designed as feedforward neural networks. In recent research, however, deep learning structures have been successfully applied to a recurrent neural network (RNN). As an example, there are cases in which the deep neural network structure was applied to the field of language modeling. A convolutional neural network (CNN) has been well applied to the field of computer vision, and successful application cases have been well documented. Furthermore, in recent years, the convolutional neural network has been applied to the field of acoustic modeling for automatic speech recognition (ASR), and it is evaluated that the convolutional neural network has been more successfully applied than existing models. The deep neural network may be trained using a standard error back-propagation algorithm. At this time, weights may be updated through stochastic gradient descent using the following equation.

The present disclosure proposes a healthcare apparatus capable of predicting information related to a living body (a heart rate, a sleep state, such as sleep apnea, a wake/sleep state, etc.) and a stress index of the living body based only on information of a facial image in a contactless state using a predetermined trained algorithm model. A health monitoring method of predicting PPG and a respiratory rate (RR) based on a facial image in a contactless manner may be a health monitoring method that is suitable for a subject who needs continuous health monitoring, such as an infant or an adult having a disease, such as glycosuria, and that is safe in an environment in which contagious COVID-19 is prevalent. The present disclosure proposes a method of predicting the heart rate, the stress index, whether the subject is in a sleep apnea state, or the wake/sleep state of a subject using a remote PPG (rPPG) signal and a ballistocardiogram (BCG) signal. Hereinafter, ballistocardiogram (BCG) and photoplethysmography (PPG), as biometric information to be used in the present disclosure, acquired by sensing a human body will be described briefly first.

Photoplethysmogram (PPG) sensor

A photoplethysmography (PPG) sensor will be described as an example of a heart rate sensor. PPG sensor is short for photoplethysmography sensor. Photoplethysmography (PPG) is a pulse wave measurement method that measures the flow rate of blood in a blood vessel using optical characteristics of living tissue in order to check an activity state of the heart or the heart rate. The pulse wave, which is a pulsating waveform generated in the heart while undulating, is measurable through a change in blood flow rate generated according to relaxation and contraction of the heart, i.e. a change in volume of a blood vessel. In photoplethysmography, which is a method of measuring a pulse wave using light, a change in optical properties, such as reflection, absorption, and a transmission rate, of living tissue generated at the time of volume change is sensed and measured by an optical sensor, whereby it is possible to measure pulsation. This method is capable of performing non-invasive pulse measurement, is widely used due to merits thereof, such as miniaturization and convenience in use, and may be used as a biosignal sensor in a wearable device.

Ballistocardiogram (BCG) sensor

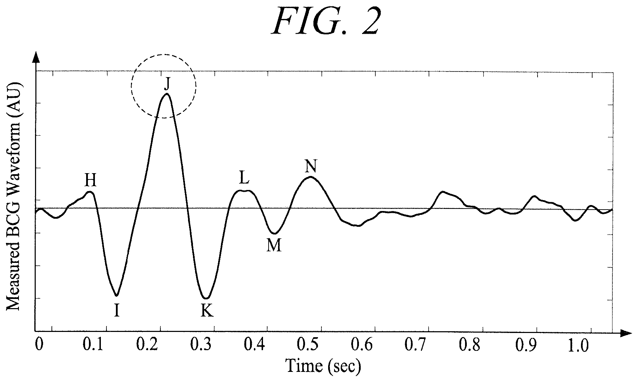

FIG. 2 is a view illustrating a ballistocardiogram (signal) waveform.

The moment blood discharged from a ventricle of the heart during a cardiac cycle passes through the aorta, the blood transmits reaction to our bodies. A signal measuring vibration (ballistic trajectory) due to a change in blood flow in the heart and the blood vessel related thereto is called ballistocardiogram (BCG). Ballistocardiogram means a signal measuring a ballistic trajectory due to a change in blood flow in the heart and the blood vessel according to contraction and relaxation of the heart, and is an index indicating the activity state of the heart, similarly to an electrocardiogram.

Ballistocardiogram is an index indicating the activity state of the heart, similarly to electrocardiogram, and it is known that ballistocardiogram includes information about cardiac output and information about reflux and abnormal blood flow due to damage to a myocardial function. Consequently, this biosignal has the potential to be clinically utilized, such as evaluation in function of the heart, diagnosis of heart disease (cardiomyopathy), checking of treatment effects, and observation of the degree of recovery. A ballistocardiogram signal may be measured using an acceleration sensor, a load cell sensor, a PVDF film sensor, or an EMFi sensor. Since it is not necessary to attach an electrode to the body when these sensors are used, it is possible to measure a signal in an unconstrained/unconscious state, and the sensors may be usefully utilized in health monitoring for a long time or during everyday life.

As shown in FIG. 2, in a ballistocardiogram signal, a heart rate pattern is expressed by peaks H, I, J, and K, and a part that is admitted as a real heat rate is peak J. In the ballistocardiogram signal, the heart rate pattern appears in various forms due to noise, environment, and personal influence. In general, peak I shows a noticeably big difference depending on environment, measurement conditions, and individual differences, and peaks H and J have nonuniform sizes. Also, when peak I is small, only one of peaks H and J may appear in a large form.

A remote measurement method will be described in brief.

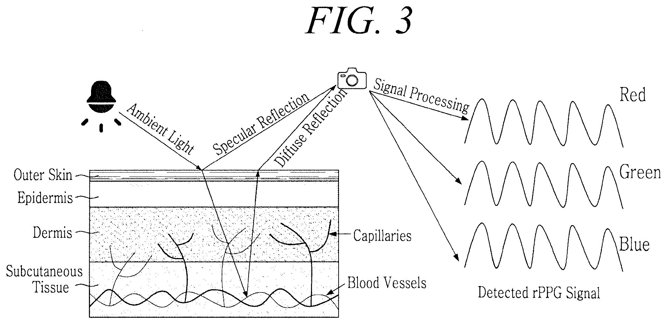

FIG. 3 is an illustrative view provided to explain remote PPG (rPPG).

It is possible to remotely measure a heart rate and heart rate variability using remote PPG. As shown in FIG. 3, video recording may be performed using a high-resolution camera in order to remotely measure a heart rate, etc. This may be useful for various physical, health, and emotional monitoring on a driver, an elder, or an infant. The remote PPG is contactless measurement, although the remote PPG is identical in principle to PPG. As contrast between specular reflection and diffuse reflection, a change in red, green, and blue light reflection on the skin is measured. The specular reflection is pure light reflection from the skin. The diffuse reflection is reflection remaining due to changes in absorption and scattering by skin tissue depending on the amount of blood. This is the principle using the fact that hemoglobin reflects red light and absorbs green light. FIG. 3 illustrates red, green, and blue light waveforms detected after signal processing is performed using a remote PPG technique, and it can be seen that rPPG waveforms detected for these kinds of light are different from each other. Utilization of a remote measurement technique for remotely monitoring biometric information is very high in that it is possible to predict biometric information using only information of a face in a contactless manner in an environment in which infant monitoring or contactless monitoring, for example contagious disease monitoring, such as COVID-19 monitoring, is required. A facial image is acquired using ambient light around a subject as a light source.

Multi-Task Learning (MTL)

Many attempts to predict biometric information using a deep neural network and machine learning have been made. In implementing a mobile medical system, multi-task learning (MTL) is an important approach to perform various tasks using limited resources. A PPG signal and a respiratory rate signal are simultaneously extracted from a facial video stream using MTL. The present invention proposes a complex-value-based multi-task learning (MTL) algorithm model that simultaneously processes video streams. Two facial areas are constituted by complex number data that are simultaneously processed in a neural network architecture having complex values. Through this complex process, the PPG signal and the respiratory rate signal may be more efficiently and accurately extracted than an actual-value-based single-task learning algorithm.

Multi-task learning (MTL) is a model learning method of performing prediction by simultaneously learning various tasks, e.g. two or more tasks, through a shared layer. As relevant tasks are simultaneously learned, learned representation may be shared, and therefore tasks having good representation may be helpful in model learning. Useful information acquired through learning may have a good influence on other tasks, thereby contributing to becoming a better model. In addition, many tasks may be simultaneously predicted, whereby it is possible to achieve training to a generalized model more robust to overfitting, and two existing tasks are combined into one model, whereby it is possible to lighten the model, which is more advantageous in application to a mobile device, such as a smartphone.

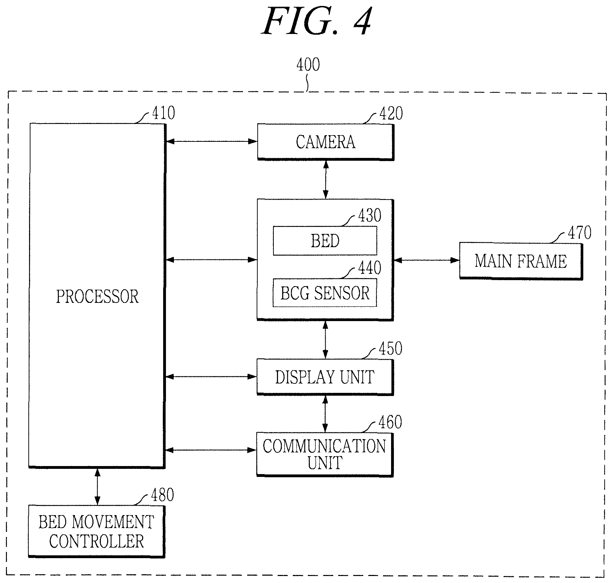

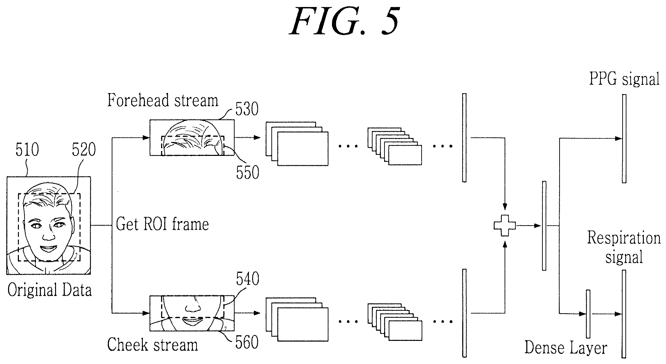

FIG. 4 is a block diagram provided to explain the construction of a healthcare apparatus 400 according to an embodiment, and FIG. 5 is a view provided to explain output of a PPG signal and a respiratory rate (RR) signal from an MTL algorithm learning model using a remote PPG technique.

Referring to FIG. 4, the healthcare apparatus 400 may include a processor 410, a camera 420, a bed 430, a BCG sensor 440, a display unit 450, a communication unit 460, a main frame 470, and a bed motion controller 480.

The camera 420 captures an image of the face of a subject (e.g. an infant lying on the bed 430). The camera 420 may transmit the captured facial image of the subject to the processor 410 through the communication unit 460. The processing 410 may acquire the facial image of the subject. Here, the captured facial image of the subject may be a color image, such as an RGB image.

Referring to FIG. 5, the processor 410 may acquire original data 510 having the captured facial image of the subject, and may detect the facial image of the subject from the original data 510. The processor 410 may acquire region-of-interest frames 530 and 540 from the facial image of the subject, and may detect a forehead area 430 and a cheek area 560 from the region-of-interest frames 530 and 540, respectively. The processor 410 may learn the facial image using a convolutional neural network (CNN) or a Siamese neural network (SNN), which is a deep learning network used in image processing, to predict a PPG signal and a respiratory rate signal. In the present invention, it is preferable for the multi-task learning algorithm learning model to use the Siamese neural network (SNN). The processor 410 repeatedly applies a filter (kernel) to all areas of the forehead image and the cheek image extracted from the facial image 520 in order to find and learn a pattern. The reason that the forehead and the cheek are extracted is that the difference between two regions of the body due to the time difference in blood rising from the heart is utilized as meaningful information in order to train the model.

Each of the image of the detected forehead area and the image of the cheek area is a color image. Hereinafter, the image 550 of the forehead area will be referred to as a first color image, and the image 560 of the cheek area will be referred to as a second color image, for convenience of description. The processor 410 converts the first color image and the second color image into black and white images, and acquires a first black and white image from the first color image and a second black and white image from the second color image. After conversion into the black and white images, the processor 410 learns through the MTL (e.g. multi-task Siamese network) to predict a PPG signal and a respiratory rate signal.

As shown in FIG. 5, the processor 410 converts the first color image of the forehead area and the second color image of the cheek area into a first black and white image and a second black and white image, respectively, and inputs the converted images to a predetermined trained multi-task learning algorithm model using the Siamese neural network (SNN). The processor 410 applies the first black and white image and the second black and white image to the predetermined trained multi-task learning algorithm model to output a remote PPG signal waveform (which may be called a PPG signal waveform output using an rPPG technique) and a respiratory rate (RR) waveform. As described above, it is preferable for the processor 410 to convert the color images of the forehead area and the cheek area into black and white images, respectively, and to input the black and white images to the predetermined trained algorithm model when a predetermined time is daytime or when the level of the ambient light is a predetermined level or more. When the predetermined time is daytime or when the level of the ambient light is the predetermined level or more, the processor 410 may perform control such that the camera 420 photographs the face of the subject to acquire a color image.

When the predetermined time is nighttime or when the level of the ambient light is less than the predetermined level, on the other hand, the processor 410 may perform control such that the camera 420 or a separate camera configured to capture an infrared image photographs the face of the subject to acquire an infrared (IR) image. When the predetermined time is nighttime or when the level of the ambient light is less than the predetermined level, the camera 420 may photograph the face of the subject to acquire an infrared (IR) image, and the processor 410 may detect a region of interest (ROI) corresponding to the face from the infrared image, and may acquire a first image of the forehead area and a second image of the cheek area from the detected region of interest. In addition, the processor 410 may apply the first image and the second image acquired from the infrared image to the predetermined trained multi-task learning algorithm model in order to output a remote photoplethysmography (rPPG) signal waveform of the subject. Subsequently, the processor 410 may calculate a first heart rate from the sensed BCG signal waveform, may calculate a second heart rate from the output remote PPG signal waveform, and may output the heart rate of the subject based on the first heart rate and the second heart rate.

As described above, the processor 410 may select whether to acquire the face of the subject as a color image or an infrared image depending on the time zone or the ambient light. The processor 410 may convert the color image into a black and white image according to selection based on the time zone or the ambient light, and may input the black and white image to the predetermined trained algorithm (MTL algorithm) model so as to be applied thereto, or may input an image acquired from the infrared image to the predetermined trained algorithm model so as to be applied thereto.

The healthcare apparatus 400 may include the bed 430, on which the subject (e.g. an infant) may lie. The BCG sensor 440 may be attached to the inside of a cover of the bed 430. Since the BCG sensor 440 is provided at an inner surface of the cover of the bed 430, it is possible to sense a ballistocardiogram from the back or the flank of the subject when the subject lies on the bed.

The processor 410 may calculate a heart rate from the ballistocardiogram signal waveform acquired from the BCG sensor 440, may acquire a PPG signal waveform from the predetermined trained MTL algorithm model, and may calculate a heart rate from the acquired PPG signal waveform. Here, the heart rate calculated from the ballistocardiogram signal waveform is referred to as a first heart rate, and the heart rate calculated from the PPG signal waveform is referred to as a second heart rate. The first heart rate may be calculated as the number of peaks J in the BCG signal waveform per unit time (e.g. 1 minute), and the second heart rate may be calculated as the number of peaks in the rPPG signal waveform per unit time (e.g. 1 minute). The processor 410 may output the heart rate of the subject based on the first heart rate calculated from the ballistocardiogram signal waveform and the second heart rate calculated from the PPG signal waveform acquired using the remote PPG technique. As an example, the processor 410 may output the average of the first heart rate and the second heart rate.

In addition, the processor 410 may calculate heart rate variability (HRV) from the ballistocardiogram signal waveform. As an example, the heart rate variability may be calculated by Mathematical Expression 1 below. Here, heart rate variability calculated from the ballistocardiogram signal waveform is referred to as first heart rate variability.

[Mathematical Expression 1]

$$BCG\,HRV = \frac{Jpeak\,(n+1) - Jpeak\,(n)}{SamplingRate}$$

The processor 410 may calculate heart rate variability from the PPG signal waveform from the rPPG technique based on Mathematical Expression 2 below. Here, heart rate variability calculated from the PPG signal waveform is referred to as second heart rate variability.

[Mathematical Expression 2]

$$rPPG\,HRV = \frac{peak\,(n+1) - peak\,(n)}{SamplingRate}$$

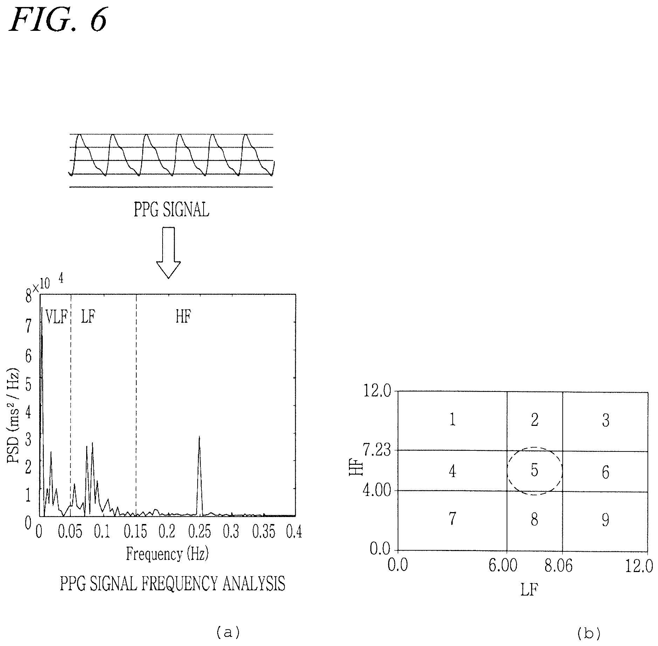

FIG. 6A and FIG. 6B are views illustrating PPG signal frequency analysis and LF score/HF score distribution for calculation of a stress index of the subject.

It is necessary for the processor 410 to perform PPG signal waveform frequency analysis and BCG signal waveform frequency analysis in order to calculate a stress index. As an example, PPG signal waveform frequency analysis is shown in FIG. 6A. The processor 410 may calculate a first stress index (score) of the subject based on the first heart rate variability, and may calculate a second stress index of the subject based on the second heart rate variability. Here, a method of calculating the first stress index and the second stress index is performed as represented by Mathematical Expression 3 below.

[Mathematical Expression 3]

$$LF = \ln \int_{0.04}^{0.15} (PSD\ of\ HRV)df\ (\text{msec}^2)$$

$$LF = \ln \int_{0.15}^{0.4} (PSD\ of\ HRV)df\ (\text{msec}^2)$$

$$LF score = \begin{cases} \dfrac{LF}{6.00} & (0 \le LF \le 6.00) \\ 1 & (6.00 < LF \le 8.06) \\ 1 - 0.5\dfrac{LF - 8.06}{12 - 8.06} & (8.06 < LF \le 12) \end{cases}$$

$$HF score = \begin{cases} \dfrac{HF}{4.00} & (0 \le HF \le 4.00) \\ 1 & (4.00 < HF \le 7.23) \\ 1 - 0.5\dfrac{HF - 7.23}{12 - 7.23} & (7.23 < HF \le 12) \end{cases}$$

$$Stress\ measurement\ score = 100 \times \left(\frac{2}{3} LF\ score + \frac{1}{3} HF\ score\right)$$

In Mathematical Expression 3, a signal of a sympathetic nervous system LF and a signal of a parasympathetic nervous system HF are calculated and converted into a stress index.

A description will be given with reference to Mathematical Expression 3 above and FIG. 6A. LF (sympathetic nerve) is an integral value in a power spectral density graph of heart rate variability at a low frequency of 0.04 to 0.15 Hz. HF (parasympathetic nerve) is an integral value in a power spectral density graph of heart rate variability at a high frequency of 0.15 to 0.40 Hz. For a stress index (score), the natural logarithm is taken for LF power and HF power, and LF score and HF score are calculated depending on the range thereof, and conversion into a stress index is performed. The stress score is a score indicating the distance from a fifth area, which is normal.

The processor 410 may calculate the first stress index (score) of the subject based on Mathematical Expression 1 above and Mathematical Expression 3 above, and may calculate the second stress index of the subject based on Mathematical Expression 2 above and Mathematical Expression 3 above. The processor 410 may output the stress index of the subject based on the first stress index and the second stress index. As an example, the processor 410 may output the average of the first and second stress indices as the stress index of the subject.

The processor 410 also outputs a respiratory rate (RR) signal waveform based on the predetermined trained multi-task learning algorithm model using the Siamese neural network (SNN), in addition to the PPG signal waveform. The processor 410 also performs a subject health monitoring function, such as a function of determining whether the subject (e.g. an infant) is in a sleep apnea state, based on the output respiratory rate signal waveform. The processor 410 may perform control such that the output respiratory rate signal waveform, the respiratory rate, etc. are displayed on the display unit 450 so as to be seen by a person who monitors the subject. In addition, the processor 410 may perform control such that the output heart rate, the output stress index, and the output (or calculated) respiratory rate are displayed on the display unit 450, whereby the health state of the subject is monitored from the outside.

The communication unit 460 may periodically or aperiodically transmit information, such as the output heart rate, the output stress index, and the output respiratory rate, to a user's terminal that performs health monitoring of the subject or a server through Wi-Fi, Bluetooth, etc. Aperiodic transmission is performed only when at least one of the output heart rate, the output stress index, and the output respiratory rate exceeds a predetermined critical value. As an example, only when a determination is made that the output respiratory rate indicates a sleep apnea state, the communication unit 460 may transmit the output respiratory rate to a linked terminal. The user may periodically or aperiodically receive health monitoring information of the subject through their linked terminal in order to check the health state of the subject.

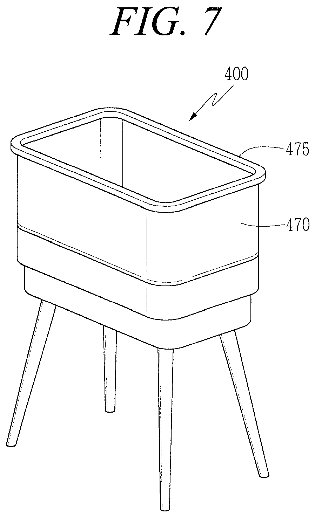

FIG. 7 is a view illustrating the healthcare apparatus 400.

Referring to FIG. 7, the main frame 470 of the healthcare apparatus 400 may include a lower support portion configured to support the bed 430 from below and a side support portion 475 disposed so as to surround the bed 430, the side support portion 475 serving as a guard configured to prevent the subject from falling from the bed 430. The camera 420 may be located at the side support portion 475 in order to photograph the face of the subject. A moving member configured to allow the camera 420 to be movable in order to track the face of the subject as the subject moves in a state of lying on the bed 430 may be provided at the side support portion 475. The camera 420 may photograph the face of the subject while moving from the side support portion 475 of the main frame 470 in consideration of the supine position of the subject and the direction in which the subject lies down.

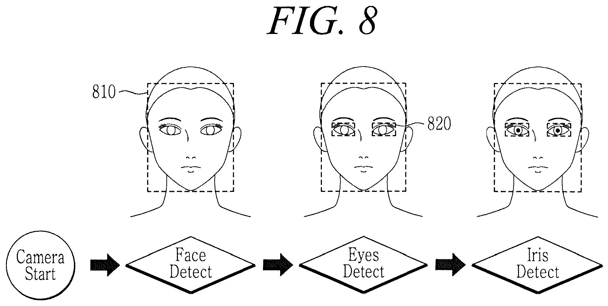

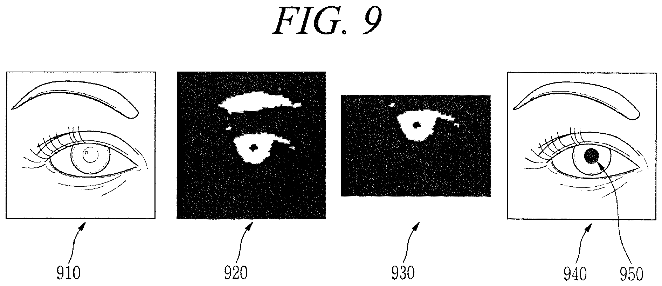

FIG. 8 is a view illustrating a step of detecting the iris of the subject in the healthcare apparatus 400, and FIG. 9 is a view describing in detail a method of detecting the pupil (iris) of the subject in the healthcare apparatus 400.

Referring to FIG. 8, the camera 420 photographs the face of the subject. The processor 410 detects an image 810 of a facial area of the subject, which is a region of interest, and extracts images 820 of two eye areas of the subject from the detected facial image 810. The processor 410 detects two irises from the detected two eye area images 820. At this time, when the two irises are detected or recognized from the detected two eye area images 820, the processor 410 determines that the subject is in a wake state. When both the two irises are not recognized from the detected two eye area images 820 for a predetermined time, on the other hand, the processor 410 determines that the subject is in a sleep state.

Referring to FIG. 9, the processor 410 may detect an eye area from the facial image of the subject to acquire an eye area image 910. The processor 410 detects a pupil from the acquired eye area image 910 to acquire a pupil area image 920, and removes an eyebrow and noise from the eye area image 910 to acquire a pupil image 930. The processor 410 may detect an iris 950 based on the pupil image 930, and may display the detected iris 950 in an eye area image 940.

For the sake of description, only one eye is shown in FIG. 9; however, the camera 420 and the processor 410 perform image capturing and image processing on two eyes of the subject.

Upon determining that the subject (e.g. an infant) is in a wake state, the processor 410 may control the bed movement controller 480 such that a bounce function that is being performed by the bed 430 is maintained. The bed movement controller 480 is provided at a lower part of the bed 430 to control horizontal and vertical movements of an upper plate of the bed 430 under control of the processor 410, whereby the bounce function of the bed 430 is performed. Upon determining that the subject is in a sleep state, on the other hand, the processor 410 may control the bed movement controller 480 such that the horizontal and vertical movements of the upper plate of the bed 430 are controlled, whereby the bounce function is slowly stopped.

Conventionally, the position of eyes is detected using brightness around the eyes and the position of eyebrows, whereby there is a problem in that other areas of the face are incorrectly recognized as the eyes. In the present invention, however, the processor 410 does not find the position of the eyes of the subject but detects irises from the two eye area images 820, checks whether the irises are recognized, and determines whether the subject is in a wake state or a sleep state, and therefore accuracy in checking the wake/sleep state of the subject is greatly improved.

The embodiments described above are predetermined combinations of elements and features of the present disclosure. Each element or feature must be considered to be optional unless explicitly mentioned otherwise. Each element or feature may be implemented in a state of not being combined with another element or feature. In addition, some elements and/or features may be combined to constitute an embodiment of the present invention. The sequence of operations described in the embodiments of the present invention may be changed. Some elements or features in a certain embodiment may be included in another embodiment, or may be replaced with corresponding elements or features in another embodiment.

The processor 410 may be implemented by hardware, firmware, software, or a combination thereof. When an embodiment is implemented using hardware, application specific integrated circuits (ASICs), digital signal processors (DSPs), digital signal processing devices (DSPDs), programmable logic devices (PLDs), or field programmable gate arrays (FPGAs), which are configured to perform the present invention, may be provided in the processor 410. The present disclosure may be implemented as a computer-readable recording medium having a program for performing the monitoring method for healthcare according to the present invention in a computer recorded therein.

Those skilled in the art will appreciate that the present invention may be embodied in other specific forms than those set forth herein without departing from the scope of the claims. The above description is therefore to be construed in all aspects as illustrative and not restrictive. The scope of the invention should be determined by the appended claims.

[Industrial Applicability]

A digital healthcare device for measuring heart rate using a remote PPG method is available for industrial use.

**Claims**

1. A digital healthcare apparatus (400) comprising:

   a processor (410) configured to determine to obtain a face of a subject as a color image based on a predetermined time being daytime or a level of ambient light being more than a predetermined level, or as an infrared (IR) image based on a predetermined time being nighttime or a level of ambient light being less than a predetermined level; and
   a camera (420) configured to obtain a facial image by photographing a face of the subject based on the determination of the processor;
   wherein the processor is configured:

   to detect a region of interest (ROI) corresponding to the face in the facial image,
   to obtain a first image for a first area and a second image for a second area in the detected ROI, the first and the second images being acquired from the obtained image, and
   to output a remote photoplethysmography (rPPG) signal waveform of the subject and a respiratory rate signal waveform as results generated from a predetermined trained algorithm model by applying the first image and the second image to the predetermined trained algorithm model performing prediction by simultaneously learning two tasks, the digital healthcare apparatus further comprising:

   ballistocardiogram (BCG) sensor (440) configured to sense a BCG signal of the subject,
   wherein the processor is configured to:

   calculate a first heart rate from the sensed BCG signal waveform,
   calculate a second heart rate from the output rPPG signal waveform, and
   to output a heart rate of the subject based on the first heart rate and the second heart rate.

2. The digital healthcare apparatus according to claim 1, wherein the output heart rate corresponds to an average of the first heart rate and the second heart rate.

3. The digital healthcare apparatus according to claim 1, wherein the predetermined trained algorithm model uses a Siamese Neural Network (SNN) of multi-task learning.

4. The digital healthcare apparatus according to claim 1, wherein the processor is configured:

   to detect two eye area images (820) in the ROI and two pupil images in the detected two eye area images, and
   to determine that the subject is in a wake state when two irises are detected and recognized from the detected two pupil images.

5. The digital healthcare apparatus according to claim 4, wherein, when both the two irises are not recognized from the detected two pupil images for a predetermined time, the processor determines that the subject is in a sleep state.

6. The digital healthcare apparatus according to claim 4, further comprising:

   a bed (430) configured to allow an infant corresponding to the subject to lie down thereon,
   wherein the processor performs control such that a bounce function that is being performed by the bed is maintained upon determining that the subject is in the wake state.

7. The digital healthcare apparatus according to claim 5, further comprising:

a bed configured to allow the subject to lie down thereon,
wherein the processor controls vertical and horizontal movements of the bed such that a bounce function is slowly stopped upon determining that the subject is in the sleep state.

8. The digital healthcare apparatus according to claim 1, further comprising:

a main frame (470), wherein
the camera is movable on the main frame to photograph the face of the subject in consideration of a supine position of the subject and a direction in which the subject lies down.

9. The digital healthcare apparatus according to claim 1,
wherein the first are is a forehead area (430) and the second area is a cheek area (560).

10. The digital healthcare apparatus according to claim 1, wherein the processor is configured to:
determine whether the subject is in a sleep apnea state based on the calculated respiratory rate.

11. A monitoring method for healthcare of a subject using a remote photoplethysmography (rPPG) scheme, the monitoring method comprising:

determining to obtain a face of the subject as a color image based on a predetermined time being daytime or a level of ambient light being more than a predetermined level, or as an infrared (IR) image based on a predetermined time being nighttime or a level of ambient light being less than a predetermined level; and
obtaining a facial image by photographing a face of the subject based on the determination of the processor;
detecting a region of interest (ROI) corresponding to the face in the facial image;
obtaining a first image for a first area and a second image for a second area in the detected ROI, the first and the second images being acquired from the obtained image; and
outputting a remote photoplethysmography (rPPG) signal waveform of the subject and a respiratory rate signal waveform as results generated from a predetermined trained algorithm model by applying the first image and the second image to the predetermined trained algorithm model performing prediction by simultaneously learning two tasks, the monitoring method further comprising:

sensing a ballistocardiogram (BCG) signal of the subject;
calculating a first heart rate from the sensed BCG signal waveform;
calculating a second heart rate from the output rPPG signal waveform; and
outputting a heart rate of the subject based on the first heart rate and the second heart rate.

**Patentansprüche**

1. Eine Digitale Gesundheitsvorrichtung (400), umfassend:

einen Prozessor (410), der konfiguriert ist, zu bestimmen, ein Gesicht eines Subjekts als Farbbild zu erhalten, basierend darauf, dass eine vorgegebene Zeit Tageszeit ist oder ein Umgebungslichtniveau höher als ein vorgegebenes Niveau ist, oder als Infrarotbild (IR-Bild) zu erhalten, basierend darauf,
dass eine vorgegebene Zeit Nachtzeit ist oder ein Umgebungslichtniveau niedriger als ein vorgegebenes Niveau ist; und
eine Kamera (420), die konfiguriert ist, ein Gesichtsbild durch Fotografieren eines Gesichts des Subjekts basierend auf der Bestimmung des Prozessors zu erhalten;
wobei der Prozessor konfiguriert ist:

einen Bereich von Interesse (ROI), der dem Gesicht entspricht, in dem Gesichtsbild zu detektieren,
ein erstes Bild für einen ersten Bereich und ein zweites Bild für einen zweiten Bereich in dem detektierten ROI zu erhalten, wobei das erste und das zweite Bild aus dem erhaltenen Bild erfasst werden, und
eine Signalwellenform einer Fern-Photoplethysmographie (rPPG) des Subjekts und eine Atemfrequenz-Signalwellenform als Ergebnisse auszugeben, die von einem vorgegebenen trainierten Algorithmusmodell erzeugt werden, indem das erste Bild und das zweite Bild auf das vorgegebene trainierte Algorithmusmodell angewendet werden, das eine Vorhersage durch gleichzeitiges Lernen zweier Aufgaben durchführt,
wobei die digitale Gesundheitsvorrichtung ferner umfasst:

einen Ballistokardiogramm-Sensor (BCG-Sensor) (440), der konfiguriert ist, ein BCG-Signal des Subjekts zu erfassen,
wobei der Prozessor konfiguriert ist:

eine erste Herzfrequenz aus der erfassten BCG-Signalwellenform zu berechnen,
eine zweite Herzfrequenz aus der ausgegebenen rPPG-Signalwellenform zu berechnen, und
eine Herzfrequenz des Subjekts basierend auf der ersten Herzfrequenz und der zweiten Herzfrequenz auszugeben.

2. Die digitale Gesundheitsvorrichtung nach Anspruch 1, wobei die ausgegebene Herzfrequenz einem Mittelwert der ersten Herzfrequenz und der zweiten Herzfrequenz entspricht.

3. Die digitale Gesundheitsvorrichtung nach Anspruch 1, wobei das vorgegebene trainierte Algorithmusmodell ein siamesisches neuronales Netzwerk (SNN) des Multi-Task-Lernens verwendet.

4. Die digitale Gesundheitsvorrichtung nach Anspruch 1, wobei der Prozessor konfiguriert ist:

zwei Augenbereichsbilder (820) in dem ROI und zwei Pupillenbilder in den detektierten zwei Augenbereichsbildern zu detektieren, und
zu bestimmen, dass sich das Subjekt in einem Wachzustand befindet, wenn zwei Iriden aus den detektierten zwei Pupillenbildern detektiert und erkannt werden.

5. Die digitale Gesundheitsvorrichtung nach Anspruch 4, wobei der Prozessor bestimmt, dass sich das Subjekt in einem Schlafzustand befindet, wenn die beiden Iriden aus den detektierten zwei Pupillenbildern über eine vorgegebene Zeit hinweg nicht erkannt werden.

6. Die digitale Gesundheitsvorrichtung nach Anspruch 4, ferner umfassend:

ein Bett (430), das konfiguriert ist, einem Säugling, das dem Subjekt entspricht, zu ermöglichen, darauf zu liegen,
wobei der Prozessor eine Steuerung derart durchführt, dass eine Wippfunktion, die von dem Bett ausgeführt wird, aufrechterhalten wird, wenn bestimmt wird, dass sich das Subjekt in dem Wachzustand befindet.

7. Die digitale Gesundheitsvorrichtung nach Anspruch 5, ferner umfassend:

ein Bett, das konfiguriert ist, dem Subjekt zu ermöglichen, darauf zu liegen,
wobei der Prozessor vertikale und horizontale Bewegungen des Betts derart steuert, dass eine Wippfunktion langsam gestoppt wird, wenn bestimmt wird, dass sich das Subjekt in dem Schlafzustand befindet.

8. Die digitale Gesundheitsvorrichtung nach Anspruch 1, ferner umfassend:

einen Hauptrahmen (470), wobei
die Kamera auf dem Hauptrahmen beweglich ist, um das Gesicht des Subjekts unter Berücksichtigung einer Rückenlage des Subjekts und einer Richtung, in der das Subjekt liegt, zu fotografieren.

9. Die digitale Gesundheitsvorrichtung nach Anspruch 1,
wobei der erste Bereich ein Stirnbereich (430) ist und der zweite Bereich ein Wangenbereich (560) ist.

10. Die digitale Gesundheitsvorrichtung nach Anspruch 1, wobei der Prozessor konfiguriert ist:
basierend auf der berechneten Atemfrequenz zu bestimmen, ob sich das Subjekt in einem Schlafapnoe-Zustand befindet.

11. Ein Überwachungsverfahren für die Gesundheitsversorgung eines Subjekts unter Verwendung eines Fern-Photoplethysmographie-Schemas (rPPG-Schemas), wobei das Überwachungsverfahren umfasst:

Bestimmen, ein Gesicht des Subjekts als Farbbild zu erhalten, basierend darauf, dass eine vorgegebene Zeit Tageszeit ist oder ein Umgebungslichtniveau höher als ein vorgegebenes Niveau ist, oder als Infrarotbild (IR-Bild) zu erhalten, basierend darauf, dass eine vorgegebene Zeit Nachtzeit ist oder ein Umgebungslichtniveau niedriger als ein vorgegebenes Niveau ist; und

Erhalten eines Gesichtsbilds durch Fotografieren eines Gesichts des Subjekts basierend auf der Bestimmung des Prozessors;

Detektieren eines Bereichs von Interesse (ROI), der dem Gesicht entspricht, in dem Gesichtsbild;

Erhalten eines ersten Bilds für einen ersten Bereich und eines zweiten Bilds für einen zweiten Bereich in dem detektierten ROI, wobei das erste und das zweite Bild aus dem erhaltenen Bild erfasst werden; und

Ausgeben einer Signalwellenform einer Fern-Photoplethysmographie (rPPG) des Subjekts und einer Atemfrequenz-Signalwellenform als Ergebnisse, die von einem vorgegebenen trainierten Algorithmusmodell erzeugt werden, indem das erste Bild und das zweite Bild auf das vorgegebene trainierte Algorithmusmodell angewendet werden, das eine Vorhersage durch gleichzeitiges Lernen zweier Aufgaben durchführt,

wobei das Überwachungsverfahren ferner umfasst:

Erfassen eines Ballistokardiogrammsignals (BCG-Signals) des Subjekts;

Berechnen einer ersten Herzfrequenz aus der erfassten BCG-Signalwellenform;

Berechnen einer zweiten Herzfrequenz aus der ausgegebenen rPPG-Signalwellenform; und

Ausgeben einer Herzfrequenz des Subjekts basierend auf der ersten Herzfrequenz und der zweiten Herzfrequenz.

**Revendications**

1. Dispositif de santé numérique (400) comprenant :

un processeur (410) configuré pour déterminer d'obtenir un visage d'un sujet sous forme d'une image en couleurs en fonction d'un temps prédéterminé correspondant au jour ou d'un niveau de lumière ambiante étant supérieur à un niveau prédéterminé, ou sous forme d'une image infrarouge (IR) en fonction d'un temps prédéterminé correspondant à la nuit ou d'un niveau de lumière ambiante étant inférieur à un niveau prédéterminé ; et

une caméra (420) configurée pour obtenir une image faciale en photographiant un visage du sujet sur la base de la détermination du processeur ;

le processeur étant configuré :

pour détecter une région d'intérêt (ROI) correspondant au visage dans l'image faciale,

pour obtenir une première image pour une première zone et une seconde image pour une seconde zone dans la ROI détectée, la première image et la seconde image étant acquises à partir de l'image obtenue, et

pour délivrer un signal de télécardiogramme photopléthysmographique (rPPG) en forme d'onde du sujet et un signal de fréquence respiratoire en forme d'onde en tant que résultats générés à partir d'un modèle d'algorithme prédéterminé entraîné en appliquant la première image et la seconde image au modèle d'algorithme prédéterminé entraîné effectuant une prédiction par apprentissage simultané de deux tâches,

le dispositif de santé numérique comprenant en outre :

un capteur de balistocardiogramme (BCG) (440) configuré pour détecter un signal BCG du sujet,

le processeur étant configuré pour :

calculer une première fréquence cardiaque à partir de la forme d'onde du signal BCG détecté,

calculer une seconde fréquence cardiaque à partir de la forme d'onde du signal rPPG délivré, et

délivrer une fréquence cardiaque du sujet sur la base de la première fréquence cardiaque et de la seconde fréquence cardiaque.

2. Dispositif de santé numérique selon la revendication 1, dans lequel la fréquence cardiaque délivrée correspond à une moyenne de la première fréquence cardiaque et de la seconde fréquence cardiaque.

3. Dispositif de santé numérique selon la revendication 1, dans lequel le modèle d'algorithme prédéterminé entraîné utilise un réseau de neurones siamois (SNN) à apprentissage multitâche.

4. Dispositif de santé numérique selon la revendication 1, dans lequel le processeur est configuré :

pour détecter deux images de zone oculaire (820) dans la ROI et deux images de pupille dans les deux images de zone oculaire détectées, et

pour déterminer que le sujet est dans un état d'éveil lorsque deux iris sont détectées et reconnues à partir des deux images de pupille détectées.

**5.** Dispositif de santé numérique selon la revendication 4, dans lequel, lorsque les deux iris ne sont pas tous deux reconnus à partir des deux images de pupille détectées pendant un temps prédéterminé, le processeur détermine que le sujet est dans un état de sommeil.

**6.** Dispositif de santé numérique selon la revendication 4, comprenant en outre :
un lit (430) configuré pour permettre à un nourrisson correspondant au sujet de s'y allonger,
le processeur effectuant une commande de telle sorte qu'une fonction de balancement qui est en cours d'exécution par le lit est maintenue lorsqu'il est déterminé que le sujet est dans l'état d'éveil.

**7.** Dispositif de santé numérique selon la revendication 5, comprenant en outre :

un lit configuré pour permettre au sujet de s'y allonger,
le processeur commandant des mouvements verticaux et horizontaux du lit de telle sorte qu'une fonction de rebond est arrêtée progressivement lorsqu'il est déterminé que le sujet est dans l'état de sommeil.

**8.** Dispositif de santé numérique selon la revendication 1, comprenant en outre :
un bâti principal (470), dans lequel
la caméra est mobile sur le bâti principal afin de photographier le visage du sujet en tenant compte d'une position allongée sur le dos du sujet et d'une direction dans laquelle le sujet est allongé.

**9.** Dispositif de santé numérique selon la revendication 1,
dans lequel la première zone est une zone de front (430) et la seconde zone est une zone de joue (560).

**10.** Dispositif de santé numérique selon la revendication 1, dans lequel le processeur est configuré pour :
déterminer si le sujet est dans un état d'apnée du sommeil sur la base de la fréquence respiratoire calculée.

**11.** Procédé de surveillance pour la prise en charge de la santé d'un sujet au moyen d'un schéma de télécardiogramme photopléthysmographique (rPPG), le procédé de surveillance comprenant :

déterminer d'obtenir un visage du sujet sous forme d'une image en couleurs en fonction d'un temps prédéterminé correspondant au jour ou d'un niveau de lumière ambiante étant supérieur à un niveau prédéterminé, ou sous forme d'une image infrarouge (IR) en fonction d'un temps prédéterminé correspondant à la nuit ou d'un niveau de lumière ambiante étant inférieur à un niveau prédéterminé ; et
obtenir une image faciale en photographiant un visage du sujet sur la base de la détermination du processeur ;
détecter une région d'intérêt (ROI) correspondant au visage dans l'image faciale ;
obtenir une première image pour une première zone et une seconde image pour une seconde zone dans la ROI détectée, la première image et la seconde image étant acquises à partir de l'image obtenue ; et
délivrer un signal de télécardiogramme photopléthysmographique (rPPG) en forme d'onde du sujet et un signal de fréquence respiratoire en forme d'onde en tant que résultats générés à partir d'un modèle d'algorithme prédéterminé entraîné en appliquant la première image et la seconde image au modèle d'algorithme prédéterminé entraîné effectuant une prédiction par apprentissage simultané de deux tâches, le procédé de surveillance comprenant en outre :

détecter un signal de balistocardiogramme (BCG) du sujet ;
calculer une première fréquence cardiaque à partir de la forme d'onde du signal BCG détecté ;
calculer une seconde fréquence cardiaque à partir de la forme d'onde du signal rPPG délivré ; et
délivrer une fréquence cardiaque du sujet sur la base de la première fréquence cardiaque et de la seconde fréquence cardiaque.

# FIG. 1

FIG. 2

# FIG. 3

# FIG. 4

400

# FIG. 5

PPG signal

Forehead stream 530

510    520

Get ROI frame

550

Original Data

540

Cheek stream    560

Respiration signal

Dense Layer

# *FIG. 6*

PPG SIGNAL

PPG SIGNAL FREQUENCY ANALYSIS

(a)

(b)

FIG. 7

# FIG. 8

# FIG. 9

910    920    930    940    950

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 2016206216 A1
- US 2021201496 A1
- EP 3838128 A1
- KR 20140058573 A

**Non-patent literature cited in the description**

- Improving ROI detection in photoplethysmographic imaging with thermal cameras. **SCEBBA GAETANO et al.** 2017 39th Annual International Conference of the IEEE Engineering in Medicine and Biology Society (EMBC). IEEE, 11 July 2017

- **LIU YIMING et al.** Motion-Robust Multimodal Heart Rate Estimation Using BCG Fused Remote-PPG With Deep Facial ROI Tracker and Pose Constrained Kalman Filter. *IEEE TRANSACTIONS ON INSTRU-MENTATION AND MEASUREMENT*, 19 February 2021, vol. 70, 1-15